# EUROPÄISCHE PATENTANMELDUNG

(11) **EP 1 930 010 A1**
(43) Veröffentlichungstag der Anmeldung: **11.06.2008**
(21) Anmeldenummer: 07009373.7
(22) Anmeldetag: 10.05.2007
(51) Int. Cl.: A61K 31/565, A61K 31/57, A61P 15/12, A61P 15/18, A61P 15/00

(54) **Verwendung von Estradiolvalerat oder 17ß-Estradiol in Kombination mit Dienogest zur oralen Therapie für den Erhalt und/oder die Steigerung der weiblichen Libido**

(30) Priorität: 20.10.2006 EP 06022091
(71) Anmelder: Bayer Schering Pharma Aktiengesellschaft, 13353 Berlin (DE)
(72) Erfinder: Zeun, Susan, 10245 Berlin (DE); Zimmermann, Holger, 14612 Falkensee (DE); Parke, Susanne, 14199 Berlin (DE)
(74) Vertreter: Cramer, Eva-Maria

(57) **Zusammenfassung**

Die Erfindung betrifft die Verwendung von Estradiolvalerat oder 17β-Estradiol in Kombination mit 17α-Cyanomethyl-17-β-hydroxyestra-4,9-dien-3on (Dienogest)in einem mehrphasigen oder einphasigen kombinationspräparat zur oralen Therapie für den Erhalt und/oder Steigerung der weiblichen Libido, gegebenenfalls auch in Einheit mit einer oralen Kontrazeption.

Die Gesamtzahl der Tagesdosiseinheiten der mehrphasigen Kombination und eines pharmazeutisch unbedenklichem Placebos oder der einphasigen Kombination und gegebenenfalls der pharmazeutisch unbedenklich placebohaltigen oder der placebo-und hormonfreien Tagesdosiseinheiten entsprechen 28 Tagesdosiseinheiten.

## Beschreibung

### Technisches Gebiet

Die Erfindung betrifft die Verwendung von Estradiolvalerat oder 17β-Estradiol (Estradiol) in Kombination mit 17α-Cyanomethyl-17-β-hydroxyestra-4,9-dien-3on (Dienogest) in einem mehrphasigen oder einphasigen Kombinationspräparat zur oralen Therapie für den Erhalt und/oder Steigerung der weiblichen Libido, gegebenenfalls auch in Einheit mit einer oralen Kontrazeption.

Die Gesamzahl der Tagesdosiseinheiten der mehrphasigen Kombination und eines pharmazeutisch unbedenklichem Placebos oder der einphasigen Kombination und gegebenenfalls der pharmazeutisch unbedenklich placebohaltigen oder der placebo- und hormonfreien Tagesdosiseinheiten entsprechen 28 Tagesdosiseinheiten.

### Stand der Technik

Die weibliche Libido ist ein sehr komplexes Geschehen, auf welches zahlreiche hormonelle sowie psychosoziale Faktoren, Einfluss nehmen. Bereits seit langem bekannt, dass es mit dem Eintritt in die Wechseljahre zu einer Abnahme des sexuellen Verlangens der Frauen kommen kann. Hier wird der sinkende endogene Estradiolspiegel und verminderte Androgenspiegel als Gründe diskutiert. Weniger bekannt ist, dass die Einnahme von herkömmlichen kombinierten oralen Kontrazeptiva und sogenannten "Progestin-only-Pills (POPs) zu einem erworbenen Verlust/Verringerung der Libido kommen kann. Derartige Störungen werden unter dem Begriff "aquired OC-associated hypoactive sexual desire disorder (HSDD)" zusammengefasst. (Blitzer: Kontrazeption und Sexualität, Therapeutische Umschau, Bd 51, 1994, Heft 2 110 - 114) Die Ursache hierfür ist nicht geklärt, verschiedene Thesen werden diskutiert. Zur Zeit kann dieses beobachtete Phänomen kann bisher nicht suffizient verhindert werden. (Sex steroids and libido, The European Journal of Contraception and reproductive Health care (1997 - 253 -258)

### Darstellung der Erfindung

Der Erfindung liegt die Aufgabe zugrunde, eine Möglichkeiten zu finden zu finden, welche den Erhalt der weiblichen Libido, bzw deren Steigerung realisieren.

Die Aufgabe wird erfindungsgemäß durch die Verwendung von Estradiolvalerat oder Estradiol in Kombination mit 17α-Cyanomethyl-17-β-hydroxyestra-4,9-dien-3on (Dienogest) zur Herstellung eines mehrphasigen Kombinationspräparates oder einphasigen Kombinationspräparates zur oralen Therapie für den Erhalt und/oder Steigerung der weiblichen Libido gelöst.

Es wurde gefunden, dass überraschenderweise eine orale Kontrazeption einhergeht mit dem Erhalt bzw. der Steigerung der weiblichen Libido.

Vorteilhafterweise werden Estradiolvalerat oder Estradiol in Kombination mit 17α-Cyanomethyl-17-β-hydroxyestra-4,9-dien-3on (Dienogest) zur oralen Therapie für den Erhalt bzw. die Steigerung der weiblichen Libido, gegebenenfalls in Einheit mit einer oralen Kontrazeption eingesetzt, dabei enthaltend eine erste Phase aus 2 Tagesdosiseinheiten des Estradiolvalerat zu 3 mg oder Estradiol zu kleiner 3 mg , eine zweite Phase aus 2 Gruppen von Tagesdosiseinheiten , wobei die erste Gruppe 5 Tagesdosiseinheiten einer Kombination von 2 mg Estradiolvalerat oder Estradiol kleiner 2 mg und 2 mg Dienogest und die zweite Gruppe 17 Tagesdosiseinheiten einer Kombination von 2 mg Estradiolvalerat oder Estradiol kleiner 2 mg und 3 mg Dienogest enthält,eine dritte Phase aus 2 Tagesdosiseinheiten mit 1mg Estradiolvalerat oder Estradiol kleiner 1 mg und eine weitere Phase aus 2 Tagesdosiseinheiten an pharmazeutisch unbedenklichem Placebo zur Herstellung eines mehrphasigen Kombinationspräparates mit einer Gesamtzahl von 28 .

Auch werden vorteilhafterweise gleich oder kleiner 3 mg Estradiolvalerat oder Estradiol in Kombination mit gleich oder kleiner 2,00 mg 17α-Cyanomethyl-17-β-hydroxyestra-4,9-dien-3on (Dienogest) in einem Einphasenpräparat zur oralen Therapie für den Erhalt bzw. die Steigerung der weiblichen Libido, gegebenfalls in Einheit mit einer oralen Kontrazeption eingesetzt. Die einphasige Kombination enthält 28 oder 21,22, 23, 24, 25, 26, 27 Tagesdosiseinheiten mit konstanter Menge an Dienogest und Estradiolvalerat oder Dienogest und 17β-Estradiol und 7, 6, 5, 4, 3, 2, 1 pharmazeutisch unbedenklich placebohaltige oder placebo-und hormonfreie Tagesdosiseinheiten, so dass die Gesamtzahl an Tagesdosiseinheiten 28 entspricht.

Placebo-und hormonfreie Tagesdosiseinheiten sind pillenfreie Tage.

Denkbar ist auch, dass die nach Anspruch 1 und 3 eingesetzte Menge an Estradiolvalerat zum Teil ersetzt werden durch Ethinylestradiol, so dass die im einphasigen Kombinationspräparat eingesetzte Menge an Estrogenen nicht gleich oder kleiner 3 mg Estradiolvalerat sondern beispielsweise gleich 2 mg oder 1 mg und jeweils 10 µg Ethinylestradiol beträgt

Auch ist es vorteilhaft, das einphasige Präparat im Langzeitzyklus zu verabreichen, wobei dieser aus
- einer ersten Phase aus einer Kombination von 1,0 - 2,0 mg Dienogest und 3 mg Estradiolvalerat oder kleiner 3 mg 17β-Estradiol oder aus einer Kombination von 1,0 - 2,0 mg Dienogest und 2 mg oder 1 mg Estradiolvalerat und 10 µg Ethinylestradiol zu n x 21 Tagesdosiseinheiten mit n = 2 bis 5 mit konstanter Menge an Dienogest und Estradiolvalerat oder an Dienogest und 17β-Estradiol oder an Dienogest und Estradiolvalerat und Ethinylestradiol besteht
- eine zweite Phase nach n = 2 bis 5 aus 7 pillenfreien, d.h. placebo- und hormonfreien oder aus 7 placebohaltigen Tagesdosiseinheiten besteht.

Besonders vorteilhaft erweist es sich, wenn das einphasige Kombinationspräparat mit 21-27 Tagesdosiseinheiten an Dienogest und Estradilovalerat und gegebenenfalls Ethinylestradiol oder 17β-Estradiol und 1 bis 7 placebo- und hormonfreien oder aus 1 bis 7 placebohaltigen Tagesdosiseinheiten, so dass die Gesamtzahl an Tagesdosiseinheiten 28 entspricht und daran anschließend im obigen Langzeitzyklus zur Anwendung kommt.

Untersuchungen zur Wirksamkeit der beanspruchten Formulierung

Klinische Studien werden mit einer mehrphasigen Kombination von Estradiolvalerat und Dienogest oral verabreicht , bei weicher die erste Phase aus 2 Tagesdosiseinheiten des Estradiolvalerat zu 3 mg, die zweite Phase aus 2 Gruppen von Tagesdosiseinheiten besteht. Die erste Gruppe dieser zweiten Phase besteht aus 5 Tagesdosiseinheiten einer Kombination von 2 mg Estradiolvalerat und 2 mg Dienogest und die zweite Gruppe aus 17 Tagesdosiseinheiten einer Kombination von 2 mg Estradiolvalerat und 3 mg Dienogest. Die dritte Phase enthält 2 Tagesdosiseinheiten mit 1mg Estradiolvalerat und eine weitere Phase besteht aus 2 Tagesdosiseinheiten an pharmazeutisch unbedenklichem Placebo . Die gesamten Tage eines Verabreichungszyklus der mehrphasigen Kombination und des pharmazeutisch unbedenklichem Placebo entsprechen 28 Tagen (A).

In einer randomisierten, doppelt-blinden klinischen Studie wurden 800 Frauen im Alter zwischen 18 und 50 Jahren (400 in der Altersgruppe 18 bis 35 und 400 in der Altersgruppe 36 bis 50 Jahre), die ihr schriftliches Einverständnis zur Studienteilnahme gegeben hatten, mit 2 unterschiedlichen Behandlungen, behandelt.

Die Behandlung (A) entsprach der genannten Kombination

Die zweite Behandlung (B) entsprach Miranova (20 µg Ethinylestradiol und 0,1 mg Levonorgestrel).

Der Versuch umfasste einen Vorbehandlungszyklus, 7 Behandlungszyklen und einen Nachbehandlungszyklus (Follow-up-Phase).

## Patentansprüche

1. Verwendung von Estradiolvalerat oder 17β-Estradiol in Kombination mit 17α-Cyanomethyl-17-β-hydroxyestra-4,9-dien-3on (Dienogest) zur Herstellung eines mehrphasigen Kombinationspräparates oder einphasigen Kombinationspräparates zur oralen Therapie für den Erhalt und/oder Steigerung der weiblichen Libido, gegebenenfalls in Einheit mit einer oralen Kontrazeption.

2. Verwendung von Estradiolvalerat oder 17β-Estradiol in Kombination mit 17α-Cyanomethyl-17-β-hydroxyestra-4,9-dien-3on (Dienogest) nach Anspruch 1, dabei enthaltend
eine erste Phase aus 2 Tagesdosiseinheiten des Estradiolvalerat zu 3 mg oder Estradiol zu kleiner 3 mg,
eine zweite Phase aus 2 Gruppen von Tagesdosiseinheiten , wobei
die erste Gruppe 5 Tagesdosiseinheiten einer Kombination von 2 mg Estradiolvalerat oder Estradiol kleiner 2 mg und 2 mg Dienogest
und die zweite Gruppe 17 Tagesdosiseinheiten einer Kombination von 2 mg Estradiolvalerat oder Estradiol kleiner 2 mg und 3 mg Dienogest enthält,
eine dritte Phase aus 2 Tagesdosiseinheiten mit 1mg Estradiolvalerat oder Estradiol kleiner 1 mg und eine weitere Phase aus 2 Tagesdosiseinheiten an pharmazeutisch unbedenklichem Placebo
in einem mehrphasigen Kombinationspräparates mit einer Gesamtzahl von 28 Tagesdosiseinheiten zur oralen Therapie für den Erhalt und/oder die Steigerung der weiblichen Libido, gegebenenfalls in Einheit mit einer oralen Kontrazeption.

3. Verwendung von Estradiolvalerat oder 17β-Estradiol in Kombination mit 17α-Gyanomethyl-17-β-hydroxyestra-4,9-dien-3on (Dienogest) nach Anspruch 1, dabei enthaltend gleich oder kleiner 3 mg Estradiolvalerat oder Estradiol in Kombination mit 1,00 mg - 2,00 mg 17α-Cyanomethyl-17-β-hydroxyestra-4,9-dien-3on (Dienogest) in einem einphasigen Kombinationspräparat
mit einer Gesamtzahl von 28 Tagesdosiseinheiten
oder einer Gesamtzahl von 21 Tagesdosiseinheiten und 7 pharmazeutisch unbedenkliche placebohaltige oder placebo- und hormonfreie Tagesdosiseinheiten,
oder einer Gesamtzahl von 22 Tagesdosiseinheiten und 6 pharmazeutisch unbedenkliche placebohaltige oder placebo- und hormonfreie Tagesdosiseinheiten,
oder einer Gesamtzahl von 23 Tagesdosiseinheiten und 5 pharmazeutisch unbedenkliche placebohaltige oder placebo- und hormonfreie Tagesdosiseinheiten
oder einer Gesamtzahl von 24 Tagesdosiseinheiten und 4 pharmazeutisch unbedenkliche placebohaltige oder placebo- und hormonfreie Tagesdosiseinheiten
oder einer Gesamtzahl von 25 Tagesdosiseinheiten und 3 pharmazeutisch unbedenkliche placebohaltige oder placebo- und hormonfreie Tagesdosiseinheiten
oder einer Gesamtzahl von 26 Tagesdosiseinheiten und 2 pharmazeutisch unbedenkliche placebohaltige oder placebo- und hormonfreie Tagesdosiseinheiten
oder einer Gesamtzahl von 27 Tagesdosiseinheiten und 1 pharmazeutisch unbedenkliche placebohaltige oder placebo- und hormonfreie Tagesdosiseinheit zur oralen Therapie für den Erhalt und/oder die Steigerung der weiblichen Libido, gegebenenfalls in Einheit mit einer oralen Kontrazeption.
